# EUROPEAN PATENT APPLICATION

(11) **EP 4 371 563 A1**
(43) Date of publication of application: **22.05.2024**
(21) Application number: 22208319.8
(22) Date of filing: 18.11.2022
(51) Int. Cl.: A61K 31/519, A61K 31/536, A61K 45/06, A61P 35/00

(54) **COMBINATIONS OF LAROTRECTINIB AND MODERATE CYP3A4 INDUCERS**

(71) Applicant: Bayer Consumer Care AG, 4052 Basel (CH)
(72) Inventor: The designation of the inventor has not yet been filed
(74) Representative: BIP Patents

(57) **Abstract**

The invention relates to Larotrectinib and salts thereof for use in a method for treating a patient having a cancer characterized by a neurotrophic receptor kinase gene fusion, wherein the patient has received and will receive a moderate CYP3A4 inducer to improve the anti-cancer efficacy of larotrectinib and salts thereof in patients taking one or more CYP3A4 modulating drugs.

## Description

### FIELD OF THE INVENTION

The invention relates to Larotrectinib and salts thereof for use in a method for treating a patient having a cancer characterized by a neurotrophic receptor kinase gene fusion, wherein the patient has received and will receive a moderate CYP3A4 inducer to improve the anti-cancer efficacy of larotrectinib and salts thereof in patients taking one or more CYP3A4 modulating drugs.

### BACKGROUND

Larotrectinib is a tropomyosin kinase inhibitor approved in the United States and elsewhere for the treatment of cancers associated with one or more neurotrophic receptor kinase gene fusions. It is among the first tumor-agnostic therapies, meaning it is not approved for cancer in a particular tissue type but rather for any cancer in any tissue type so long as it is characterized by the specified gene fusion.

Following oral administration, larotrectinib is metabolized by CYP3A4, and as such, coadministration of a CYP3A4 modulating drug could potentially alter larotrectinib pharmacokinetics and, thereby, clinical efficacy. While strategies exist for the use of larotrectinib with strong CYP3A4 inhibitors and strong CYP3A4 inducers, methods of using larotrectinib in combination with one or more inducers or inhibitors have not been disclosed.

There remains a need for improved dosing protocols for larotrectinib in combination with a drug that moderately modulates CYP3A4 activity. There remains a need for improved dosing protocols for larotrectinib in combination with a moderate CYP3A4 inducer and/or inhibitor. There remains a need for improved methods of safely treating patients with cancer using larotrectinib when the patient is receiving one or more moderate CYP3A4 inducers and/or inhibitors.

There remains a need for uses of larotrectinib in combination with a drug that moderately modulates CYP3A4 activity. There remains a need for improved uses of larotrectinib in combination with a moderate CYP3A4 inducer and/or inhibitor. There remains a need for improved uses of larotrectinib for methods of safely treating patients with cancer when the patient is receiving one or more moderate CYP3A4 inducers and/or inhibitors.

### BRIEF DESCRIPTION OF THE FIGURES

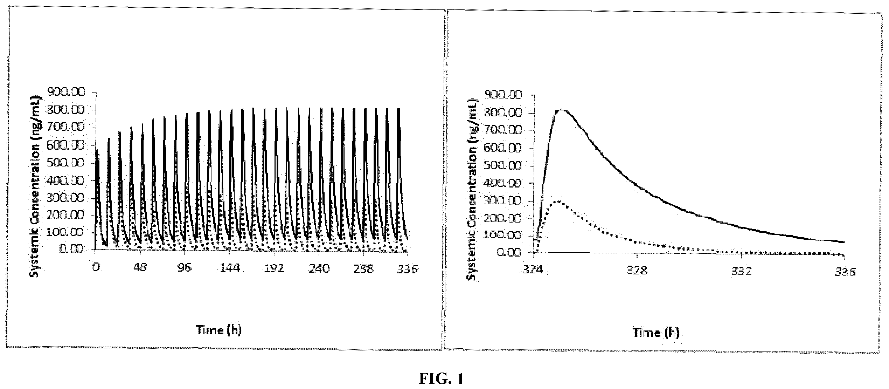

Figure 1 depicts the mean predicted plasma concentrations of larotrectinib following 14 days of dosing of larotrectinib (100 mg BID) in the absence of (solid line) and presence of efavirenz (600 mg QD) (dashed line), considering CYP3A4 inactivation. The lines are the mean data for the simulated population (n=120). Linear plots are shown for the whole dosing period (left) and the last dosing interval only (right).

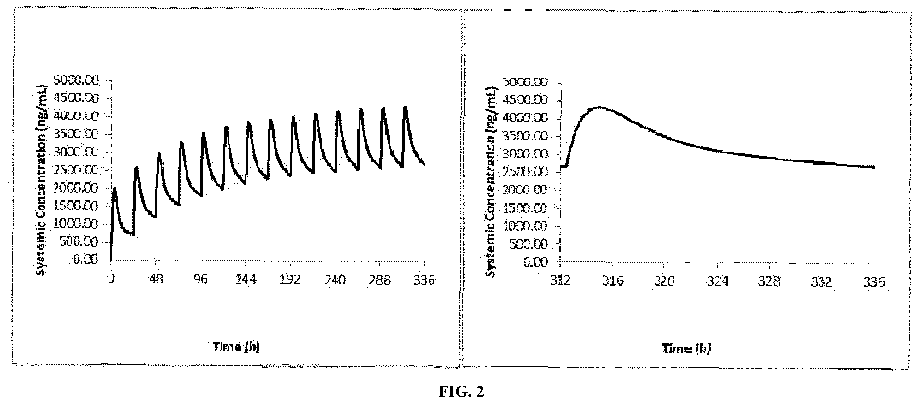

Figure 2 depicts the mean predicted plasma concentrations of efavirenz following 14 days of dosing of efavirenz (600 mg QD), considering CYP3A4 inactivation. The line is the mean data for the simulated populations (n=120). Linear plots are shown for the whole dosing period (left) and the last dosing interval only (right).

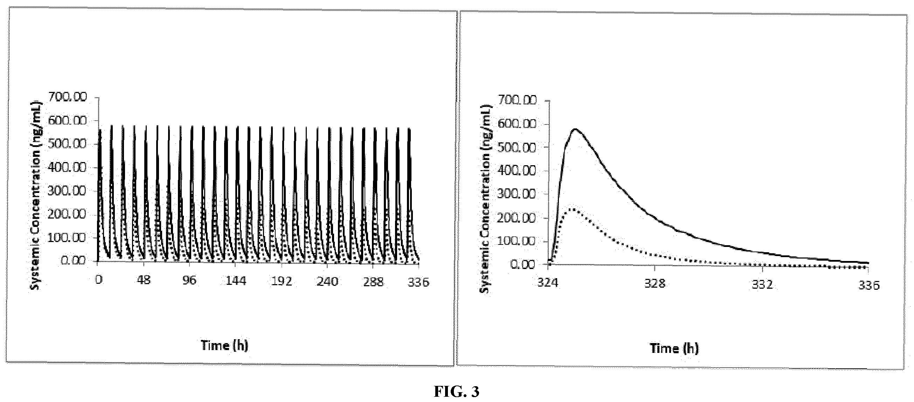

Figure 3 depicts the mean predicted plasma concentrations of larotrectinib following 14 days of dosing of larotrectinib (100 mg BID) in the absence of (solid line) and presence of efavirenz (600 mg QD) (dashed line) not considering CYP3A4 inactivation. The lines are the mean data for the simulated population (n=120). Linear plots are shown for the whole dosing period (left) and the last dosing interval only (right).

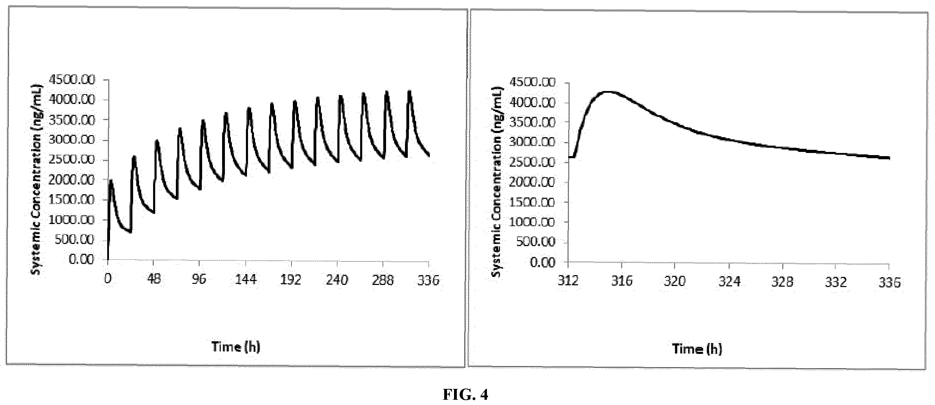

Figure 4 depicts the mean predicted plasma concentrations of efavirenz following 14 days of dosing (600 mg QD), not considering CYP3A4 inactivation. The line is the mean data for the simulated populations (n=120). Linear plots are shown for the whole dosing period (left) and the last dosing interval only (right).

### DETAILED DESCRIPTION

Before the present methods and systems are disclosed and described, it is to be understood that the methods and systems are not limited to specific synthetic methods, specific components, or particular compositions. It is also to be understood that the terminology used herein is for the purpose of describing particular embodiments only and is not intended to be limiting.

As used in the specification and the appended claims, the singular forms "a," "an," and "the" include plural referents unless the context clearly dictates otherwise. Ranges may be expressed herein as from "about" one particular value and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint and independently of the other endpoint.

"Optional" or "optionally" means that the subsequently described event or circumstance may or may not occur and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal embodiment. "Such as" is not used in a restrictive sense but for explanatory purposes.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application, including but not limited to, steps in disclosed methods. Thus, if there are a variety of additional steps that can be performed, it is understood that each of these additional steps can be performed with any specific embodiment or combination of embodiments of the disclosed methods.

Compounds disclosed herein may be provided in the form of pharmaceutically acceptable salts. Examples of such salts are acid addition salts formed with inorganic acids, for example, hydrochloric, hydrobromic, sulfuric, phosphoric, and nitric acids and the like; salts formed with organic acids such as acetic, oxalic, tartaric, succinic, maleic, fumaric, gluconic, citric, malic, methanesulfonic, p-toluenesulfonic, napthalenesulfonic, and polygalacturonic acids, and the like; salts formed from elemental anions such as chloride, bromide, and iodide; salts formed from metal hydroxides, for example, sodium hydroxide, potassium hydroxide, calcium hydroxide, lithium hydroxide, and magnesium hydroxide; salts formed from metal carbonates, for example, sodium carbonate, potassium carbonate, calcium carbonate, and magnesium carbonate; salts formed from metal bicarbonates, for example, sodium bicarbonate and potassium bicarbonate; salts formed from metal sulfates, for example, sodium sulfate and potassium sulfate; and salts formed from metal nitrates, for example, sodium nitrate and potassium nitrate.

Unless specified to the contrary, all references to dose amount refer to the amount of larotrectinib free base. By way of example, 123 mg larotrectinib sulfate (MW = 526.51 g/mol) is equivalent to 100 mg larotrectinib free base (MW = 438.44 g/mol).

As used herein, the standard dose of larotrectinib, or a pharmaceutically salt thereof, refers to the recommended dosage level for the given population. For adult patients, the standard dose is a daily dose of 200 mg. For pediatric patients, the standard dose is a daily dose of 200 mg/m².

A reduced standard dose refers to an adjusted dose resulting from a clinician's decision to lower the amount of larotrectinib in response to one or more adverse events associated with a higher dose level. For adult patients, a reduced standard dose is a daily dose that is less than 200 mg. For pediatric patients, a reduced standard dose is a daily dose that is less than 200 mg/m².

As used herein, an adverse event has the meaning defined in the Common Terminology Criteria for Adverse Events (CTCAE), published by the U.S. Department of Health and Human Services, National Institutes of Health, National Cancer Institute, herein incorporated by reference in its entirety.

A "Grade 1 adverse event," as defined by CTCAE, is a mild event, e.g., asymptomatic or mild symptoms; clinical or diagnostic observations only; intervention not indicated.

A "Grade 2 adverse event," as defined by CTCAE, is a moderate event, e.g., minimal, local, or noninvasive intervention is indicated, limiting age-appropriate activities of daily living (ADL) such as preparing meals, shopping, using the phone, managing money, etc.

A "Grade 3 adverse event," as defined by CTCAE, is a severe or medically significant event that is not immediately life-threatening; hospitalization or prolongation of hospitalization indicated; disabling or limiting self-care ADL, such as bathing, dressing, feeding self, etc. However, the patient is not bedridden.

A "Grade 4 adverse event," as defined by CTCAE, is a life-threatening event that requires urgent medical intervention.

A "Grade 5 adverse event," as defined by CTCAE, is a death related to the adverse event.

As used herein, a strong CYP3A4 inducer is a compound that causes a greater than 80% decrease in plasma AUC of sensitive index substrates.

As used herein, a moderate CYP3A4 inducer is a compound that causes between 50-80% decrease in plasma AUC of sensitive index substrates.

As used herein, "concurrently" refers to events that are temporally close in occurrence. Two drugs are said to be concurrently administered when both drugs are causing a physiological event or change in the patient at the same time. Depending on specific drugs and their duration or half-life, "concurrently receiving" can include administrations that occur on separate days. Unless specified to the contrary, when two drugs are administered concurrently, there is no limitation on whether the drugs are administered simultaneously or which drug is administered prior to the other.

Provided herein are uses of larotrectinib, a pharmaceutically acceptable salt thereof, or a combination thereof in a method for treating a patient having a cancer characterized by a neurotrophic receptor kinase gene fusion, wherein the patient has received and will receive a moderate CYP3A4 inducer. In some implementations, the uses include the steps of administering larotrectinib, a pharmaceutically acceptable salt thereof, or a combination thereof, to the patient with a 1.5-2.5 fold daily dose of larotrectinib, pharmaceutically acceptable salt thereof, or combination thereof to the patient compared to a standard dose.

In certain implementations, the patient is an adult patient. In some implementations, the patient is a pediatric patient.

In some implementations, a 2-fold daily dose of larotrectinib, pharmaceutically acceptable salt thereof, or combination thereof, is administered relative to the standard dose.

In some implementations, the larotrectinib is administered as a sulfate salt having the formula:

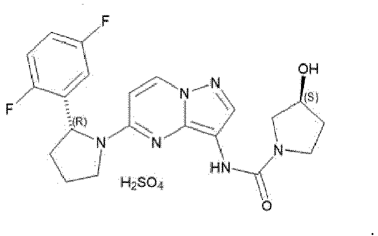

The larotrectinib sulfate may be administered in combination with other salts of larotrectinib as well as the free base. In some implementations, for example, when the larotrectinib sulfate is administered as a liquid formulation, the skilled person understands that some amount of salt exchange between the sulfate and other counterions may occur. Accordingly, in some implementations, larotrectinib is administered in a liquid formulation, wherein the formulation is prepared by combining larotrectinib sulfate with one or more further excipients.

In some implementations, the larotrectinib, pharmaceutically acceptable salt thereof, or combination thereof, is administered in a daily dose that is equivalent to 250-500 mg of the free base.

In some implementations, the larotrectinib, pharmaceutically acceptable salt thereof, or combination thereof, is administered in a daily dose that is equivalent to 400 mg of the free base.

In some implementations, the larotrectinib, pharmaceutically acceptable salt thereof, or combination thereof, is administered in two equal doses.

In some implementations, the larotrectinib, pharmaceutically acceptable salt thereof, or combination thereof, is administered with a daily dose that is equivalent to 250-500 mg/m² of the free base if the patient has a body surface area less than 1 m².

In some implementations, the larotrectinib, pharmaceutically acceptable salt thereof, or combination thereof, is administered with a daily dose that is equivalent to 400 mg/m² of the free base.

In some implementations, the use is for the treatment of a cancer characterized by one or more of the following gene fusions: ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.

In some implementations, the use is for the treatment of one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.

In some implementations, the moderate CYP3A4 inducer includes a steroid. In some implementations, the moderate CYP3A4 inducer includes a steroid selected from the group consisting of dexamethasone, progesterone, norethindrone, estradiol, and combinations thereof.
In some implementations, the moderate CYP3A4 inducer is one or more of armodafmil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, vemurafenib, zanubrutinib, or a combination thereof.

In certain implementations, the use is for the treatment of a patient having a cancer characterized by a neurotrophic receptor kinase gene fusion, wherein the patient is further diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.

In some implementations, the use is for the treatment of a patient having a cancer characterized by a neurotrophic receptor kinase gene fusion, wherein the patient is not being concurrently treated with a strong CYP3A4 inducer.

Also disclosed herein are methods of treating a patient having a cancer characterized by a neurotrophic receptor kinase gene fusion, wherein the patient is being concurrently treated with a moderate CYP3A4 inducer, by administering an increased dose of larotrectinib, pharmaceutically acceptable salt thereof, or combination thereof, to the patient, wherein the increased dose is relative to the standard dose.

In some implementations, the methods relating to treating adult patients. In some implementations, the methods relate to treating pediatric patients.

In certain implementations, the increased dose is 1.5-2.5 times greater than the standard dose. In some implementations, the increased dose is 2 times greater than the standard dose.

In some implementations, the increased dose corresponds to a daily dose of larotrectinib free base between 250-600 mg, 300-600 mg, 250-500 mg, 300-500 mg, 350-500 mg, or 350-450 mg. In certain preferred implementations, the increased dose corresponds to a daily dose of larotrectinib free base in an amount of 400 mg.

In some implementations, the increased dose corresponds to a daily dose of larotrectinib free base between 250-600 mg/m², 300-600 mg/m², 250-500 mg/m², 300-500 mg/m², 350-500 mg/m², 350-450 mg/m². In certain preferred implementations, the increased dose corresponds to a daily dose of larotrectinib free base in an amount of 400 mg/m².

In some implementations, the method includes administering the increased dose of larotrectinib, pharmaceutically acceptable salt thereof, or combination thereof within 12 hours of the patient receiving the moderate CYP3A4 inducer.

In some implementations, the method includes administering the increased dose of larotrectinib, pharmaceutically acceptable salt thereof, or combination thereof within 8 hours of the patient receiving the moderate CYP3A4 inducer, within 6 hours of the patient receiving the moderate CYP3A4 inducer, or within 4 hours of the patient receiving the moderate CYP3A4 inducer.

In some implementations, the method includes administering the increased dose of larotrectinib, pharmaceutically acceptable salt thereof, or combination thereof no more than 12 hours before the patient receives the moderate CYP3A4 inducer.

In some implementations, the method includes administering the increased dose of larotrectinib, pharmaceutically acceptable salt thereof, or combination thereof no more than 8 hours before the patient receives the moderate CYP3A4 inducer, no more than 6 hours before the patient receives the moderate CYP3A4 inducer, or no more than 4 hours before the patient receives the moderate CYP3A4 inducer.

In some implementations, the method includes administering the increased dose of larotrectinib, pharmaceutically acceptable salt thereof, or combination thereof no more than 12 hours after the patient receives the moderate CYP3A4 inducer.

In some implementations, the method includes administering the increased dose of larotrectinib, pharmaceutically acceptable salt thereof, or combination thereof no more than 8 hours after the patient receives the moderate CYP3A4 inducer, no more than 6 hours after the patient receives the moderate CYP3A4 inducer, or no more than 4 hours after the patient receives the moderate CYP3A4 inducer.

In certain implementations, the method includes the administration of the increased dose of larotrectinib, or a pharmaceutically acceptable salt thereof, to produce a larotrectinib plasma concentration (AUC_{0-inf}) that is within 50% of the plasma concentration in a patient that receives a daily dose of larotrectinib or pharmaceutically acceptable in an amount that corresponds to a daily dose of 200 mg larotrectinib free base, wherein the patient is not being concurrently treated with a moderate or strong CYP3A4 modulating drug. In some implementations, the method includes the administration of the increased dose of larotrectinib, or a pharmaceutically acceptable salt thereof, to produce a larotrectinib plasma concentration (AUC_{0-inf}) that is within 75% of the plasma concentration in a patient that receives a daily dose of larotrectinib or pharmaceutically acceptable in an amount that corresponds to a daily dose of 200 mg larotrectinib free base, wherein the patient is not being concurrently treated with a moderate or strong CYP3A4 inducer.

In certain aspects, there is provided a method of treating a patient having a cancer characterized by a neurotrophic receptor kinase gene fusion, wherein the patient is being concurrently treated with a moderate CYP3A4 inducer, by administering an increased dose of larotrectinib, pharmaceutically acceptable salt thereof, or combination thereof, wherein the increased dose does not increase the occurrence of CTCAE Grade 3 events. In some implementations, the increased dose does not increase the occurrence of CTCAE Grade 4 events.

In some implementations, the rate of adverse events is measured relative to the administration of the standard dose of larotrectinib, pharmaceutically acceptable salt thereof, or combination thereof when the patient is not receiving a moderate or strong CYP3A4 inducer.

In some implementations, the rate of adverse events is measured relative to the administration of a 200 mg daily dose of larotrectinib, pharmaceutically acceptable salt thereof, or combination thereof when the patient is not receiving a moderate or strong CYP3A4 inducer.

In some implementations, the rate of adverse events is measured relative to the administration of a 200 mg/m² daily dose of larotrectinib, pharmaceutically acceptable salt thereof, or combination thereof when the patient is not receiving a moderate or strong CYP3A4 inducer.

In some implementations, the increased dose of larotrectinib is administered as a larotrectinib sulfate salt. In some implementations, the increased dose of larotrectinib is administered as a larotrectinib sulfate salt having the formula:

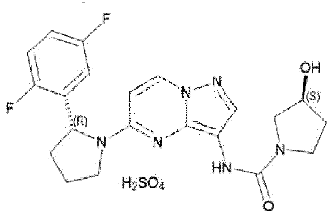

In some implementations, the larotrectinib, pharmaceutically acceptable salt thereof, or combination thereof, is administered in a capsule. In some implementations, the larotrectinib, pharmaceutically acceptable salt thereof, or combination thereof, is administered in a liquid formulation. In some implementations, the liquid formulation is prepared by combining larotrectinib sulfate with one or more suitable pharmaceutical excipients.

In some implementations, the method includes the steps of administering the increased dose in two equal doses a day. In some implementations, the doses are administered in two dosing events separated by at least 2 hours, 4 hours, 6 hours, 8 hours, or 10 hours.

In some implementations, the method includes treating a cancer characterized by a neurotrophic receptor kinase gene fusion, wherein the neurotrophic receptor kinase gene fusion is one or more of ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.

In some implementations, the method includes treating a cancer characterized by a neurotrophic receptor kinase gene fusion, wherein the cancer is one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.

In some implementations, the moderate CYP3A4 inducer includes a steroid. In certain implementations, the steroid is one or more of dexamethasone, progesterone, norethindrone, or estradiol.

In some implementations, the moderate CYP3A4 inducer includes armodafmil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, vemurafenib, zanubrutinib, or a combination thereof.

In some implementations, the patient having a cancer characterized by a neurotrophic receptor kinase gene fusion is also diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.

In some implementations, the patient is not concurrently receiving a strong CYP3A4 inducer. Exemplary strong CYP3A4 drugs include apalutamide, carbamazepine, enzalutamide, ivosidenib, lumacaftor, mitotane, phenytoin, rifampin, and St. John's wort.

### ADDITIONAL EMBODIMENTS

1. Larotrectinib, a pharmaceutically acceptable salt thereof, or a combination thereof for use in a method of safely and effectively administering Larotrectinib or a salt thereof for treating solid tumors that display a Neurotrophic Tyrosine Receptor Kinase (NTRK) gene fusion in a human patient who has taken or will take concurrently a dose of a moderate CYP3A4 inducer said method comprising:
   orally administering a 1.5-2.5 fold daily dose of Larotrectinib, pharmaceutically acceptable salt thereof, or combination thereof to the patient compared to a standard dose so as to diminish the negative effects of the moderate CYP3A4 inducer on the efficacy of the larotrectinib or a salt thereof or combination thereof when co-administered with a moderate CYP3A4 inducer.
2. Larotrectinib, a pharmaceutically acceptable salt thereof, or a combination thereof according to embodiment 1, wherein a 2 fold daily dose of larotrectinib, pharmaceutically acceptable salt thereof, or combination thereof is orally administered.
3. Larotrectinib, a pharmaceutically acceptable salt thereof, or a combination thereof according to embodiment 1 or 2, wherein the larotrectinib, pharmaceutically acceptable salt thereof, or combination thereof, comprises a sulfate salt having the formula: 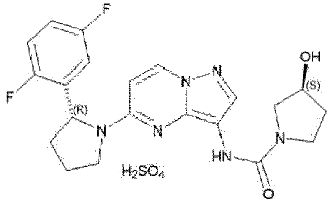
4. Larotrectinib, a pharmaceutically acceptable salt thereof, or a combination thereof according to embodiment 1, 2, or 3, wherein the larotrectinib, pharmaceutically acceptable salt thereof, or combination thereof, is administered in a liquid formulation prepared from a larotrectinib sulfate.
5. Larotrectinib, a pharmaceutically acceptable salt thereof, or a combination thereof according to any one of embodiments 1 to 4, wherein the larotrectinib, pharmaceutically acceptable salt thereof, or combination thereof is administered with a daily dose that is equivalent to 250-500 mg of the free base.
6. Larotrectinib, a pharmaceutically acceptable salt thereof, or a combination thereof according to any one of embodiments 1 to 5, wherein the larotrectinib, pharmaceutically acceptable salt thereof, or combination thereof is administered with a daily dose that is equivalent to 400 mg of the free base.
7. Larotrectinib, a pharmaceutically acceptable salt thereof, or a combination thereof according to any one of embodiments 1 to 6, wherein the larotrectinib, pharmaceutically acceptable salt thereof, or combination thereof is administered in two equal doses.
8. Larotrectinib, a pharmaceutically acceptable salt thereof, or a combination according to any one of embodiments 1 to 7, wherein the larotrectinib, pharmaceutically acceptable salt thereof, or combination thereof is administered with a daily dose that is equivalent to 250-500 mg/m² of the free base if the patient has a body surface area less than 1 m².
9. Larotrectinib, a pharmaceutically acceptable salt thereof, or a combination according to embodiment 8, wherein the larotrectinib, pharmaceutically acceptable salt thereof, or combination thereof is administered with a daily dose that is equivalent to 400 mg/m² of the free base.
10. Larotrectinib, a pharmaceutically acceptable salt thereof, or a combination thereof according to any one of embodiments 1 to 9, wherein the neurotrophic receptor kinase gene fusion comprises one or more of ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.
11. Larotrectinib, a pharmaceutically acceptable salt thereof, or a combination thereof according to any one of embodiments 1 to 10, wherein the cancer characterized by a neurotrophic receptor kinase gene fusion comprises one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.
12. Larotrectinib, a pharmaceutically acceptable salt thereof, or a combination according to any one of embodiments 1 to 11, wherein the moderate CYP3A4 inducer comprises a steroid.
13. Larotrectinib, a pharmaceutically acceptable salt thereof, or a combination according to any one of embodiments 1 to 12, wherein moderate CYP3A4 inducer comprises a steroid selected from the group consisting of dexamethasone, progesterone, norethindrone, estradiol, and combinations thereof.
14. Larotrectinib, a pharmaceutically acceptable salt thereof, or a combination according to any one of embodiments 1 to 13, wherein the moderate CYP3A4 inducer comprises armodafinil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, vemurafenib, zanubrutinib, or a combination thereof.
15. Larotrectinib, a pharmaceutically acceptable salt thereof, or a combination according to any one of embodiments 1 to 14, wherein the patient is diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.
16. Larotrectinib, a pharmaceutically acceptable salt thereof, or a combination according to any one of embodiments 1 to 15, wherein the patient is not being concurrently treated with a strong CYP3A4 inducer.

1. Larotrectinib, a pharmaceutically acceptable salt thereof, or a combination for use in a method for treating a patient having a cancer characterized by a neurotrophic receptor kinase gene fusion, wherein the patient is receiving a moderate CYP3A4 inducer, said method comprising:
   administering larotrectinib, a pharmaceutically acceptable salt thereof, or a combination thereof, to the patient with a 1.5-2.5 fold daily dose of Larotrectinib, pharmaceutically acceptable salt thereof, or combination thereof to the patient compared to a standard dose.
2. Larotrectinib, a pharmaceutically acceptable salt thereof, or a combination thereof according to embodiment 1, wherein a 2 fold daily dose of larotrectinib, pharmaceutically acceptable salt thereof, or combination thereof is administered.
3. Larotrectinib, a pharmaceutically acceptable salt thereof, or a combination thereof according to embodiment 1 or 2, wherein the larotrectinib, pharmaceutically acceptable salt thereof, or combination thereof, comprises a sulfate salt having the formula. 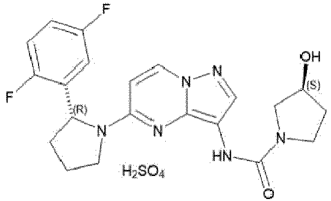
4. Larotrectinib, a pharmaceutically acceptable salt thereof, or a combination thereof according to embodiment 1, 2, or 3, wherein the larotrectinib, pharmaceutically acceptable salt thereof, or combination thereof, is administered in a liquid formulation prepared from a larotrectinib sulfate.
5. Larotrectinib, a pharmaceutically acceptable salt thereof, or a combination thereof according to any one of embodiments 1 to 4, wherein the larotrectinib, pharmaceutically acceptable salt thereof, or combination thereof is administered with a daily dose that is equivalent to 250-500 mg of the free base.
6. Larotrectinib, a pharmaceutically acceptable salt thereof, or a combination thereof according to any one of embodiments 1 to 5, wherein the larotrectinib, pharmaceutically acceptable salt thereof, or combination thereof is administered with a daily dose that is equivalent to 400 mg of the free base.
7. Larotrectinib, a pharmaceutically acceptable salt thereof, or a combination thereof according to any one of embodiments 1 to 6, wherein the larotrectinib, pharmaceutically acceptable salt thereof, or combination thereof is administered in two equal doses.
8. Larotrectinib, a pharmaceutically acceptable salt thereof, or a combination according to any one of embodiments 1 to 7, wherein the larotrectinib, pharmaceutically acceptable salt thereof, or combination thereof is administered with a daily dose that is equivalent to 250-500 mg /m² of the free base if the patient has a body surface area less than 1 m².
9. Larotrectinib, a pharmaceutically acceptable salt thereof, or a combination according to embodiment 8, wherein the larotrectinib, pharmaceutically acceptable salt thereof, or combination thereof is administered with a daily dose that is equivalent to 400 mg / m² of the free base.
10. Larotrectinib, a pharmaceutically acceptable salt thereof, or a combination thereof according to any one of embodiments 1 to 9, wherein the neurotrophic receptor kinase gene fusion comprises one or more of ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.
11. Larotrectinib, a pharmaceutically acceptable salt thereof, or a combination thereof according to any one of embodiments 1 to 10, wherein the cancer characterized by a neurotrophic receptor kinase gene fusion comprises one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.
12. Larotrectinib, a pharmaceutically acceptable salt thereof, or a combination according to any one of embodiments 1 to 11, wherein the moderate CYP3A4 inducer comprises a steroid.
13. Larotrectinib, a pharmaceutically acceptable salt thereof, or a combination according to any one of embodiments 1 to 12, wherein moderate CYP3A4 inducer comprises a steroid selected from the group consisting of dexamethasone, progesterone, norethindrone, estradiol, and combinations thereof.
14. Larotrectinib, a pharmaceutically acceptable salt thereof, or a combination according to any one of embodiments 1 to 13, wherein the moderate CYP3A4 inducer comprises armodafinil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, vemurafenib, zanubrutinib, or a combination thereof.
15. Larotrectinib, a pharmaceutically acceptable salt thereof, or a combination according to any one of embodiments 1 to 14, wherein the patient is diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.
16. Larotrectinib, a pharmaceutically acceptable salt thereof, or a combination according to any one of embodiments 1 to 15, wherein the patient is not being concurrently treated with a strong CYP3A4 inducer.

1. Larotrectinib, a pharmaceutically acceptable salt thereof, or a combination thereof for use in a method for treating a patient having a cancer characterized by a neurotrophic receptor kinase gene fusion, wherein the patient has received or will receive a moderate CYP3A4 inducer, said method comprising:
   administering larotrectinib, a pharmaceutically acceptable salt thereof, or a combination thereof, to the patient with a 1.5-2.5 fold daily dose of Larotrectinib, pharmaceutically acceptable salt thereof, or combination thereof to the patient compared to a standard dose.
2. Larotrectinib, a pharmaceutically acceptable salt thereof, or a combination thereof according to embodiment 1, wherein a 2 fold daily dose of larotrectinib, pharmaceutically acceptable salt thereof, or combination thereof is administered.
3. Larotrectinib, a pharmaceutically acceptable salt thereof, or a combination thereof according to embodiment 1 or 2, wherein the larotrectinib, pharmaceutically acceptable salt thereof, or combination thereof, comprises a sulfate salt having the formula. 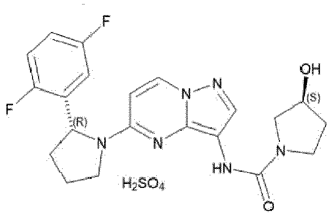
4. Larotrectinib, a pharmaceutically acceptable salt thereof, or a combination thereof according to embodiment 1, 2 or 3, wherein the larotrectinib, pharmaceutically acceptable salt thereof, or combination thereof, is administered in a liquid formulation prepared from a larotrectinib sulfate.
5. Larotrectinib, a pharmaceutically acceptable salt thereof, or a combination thereof according to any one of embodiments 1 to 4, wherein the larotrectinib, pharmaceutically acceptable salt thereof, or combination thereof is administered with a daily dose that is equivalent to 250-500 mg of the free base.
6. Larotrectinib, a pharmaceutically acceptable salt thereof, or a combination thereof according to any one of embodiments 1 to 5, wherein the larotrectinib, pharmaceutically acceptable salt thereof, or combination thereof is administered with a daily dose that is equivalent to 400 mg of the free base.
7. Larotrectinib, a pharmaceutically acceptable salt thereof, or a combination thereof according to any one of embodiments 1 to 6, wherein the larotrectinib, pharmaceutically acceptable salt thereof, or combination thereof is administered in two equal doses.
8. Larotrectinib, a pharmaceutically acceptable salt thereof, or a combination according to any one of embodiments 1 to 7, wherein the larotrectinib, pharmaceutically acceptable salt thereof, or combination thereof is administered with a daily dose that is equivalent to 250-500 mg/m² of the free base if the patient has a body surface area less than 1 m².
9. Larotrectinib, a pharmaceutically acceptable salt thereof, or a combination according to embodiment 8, wherein the larotrectinib, pharmaceutically acceptable salt thereof, or combination thereof is administered with a daily dose that is equivalent to 400 mg/m² of the free base.
10. Larotrectinib, a pharmaceutically acceptable salt thereof, or a combination thereof according to any one of embodiments 1 to 9, wherein the neurotrophic receptor kinase gene fusion comprises one or more of ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.
11. Larotrectinib, a pharmaceutically acceptable salt thereof, or a combination thereof according to any one of embodiments 1 to 10, wherein the cancer characterized by a neurotrophic receptor kinase gene fusion comprises one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.
12. Larotrectinib, a pharmaceutically acceptable salt thereof, or a combination according to any one of embodiments 1 to 11, wherein the moderate CYP3A4 inducer comprises a steroid.
13. Larotrectinib, a pharmaceutically acceptable salt thereof, or a combination according to any one of embodiments 1 to 12, wherein moderate CYP3A4 inducer comprises a steroid selected from the group consisting of dexamethasone, progesterone, norethindrone, estradiol, and combinations thereof.
14. Larotrectinib, a pharmaceutically acceptable salt thereof, or a combination according to any one of embodiments 1 to 13, wherein the moderate CYP3A4 inducer comprises armodafinil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, vemurafenib, zanubrutinib, or a combination thereof.
15. Larotrectinib, a pharmaceutically acceptable salt thereof, or a combination according to any one of embodiments 1 to 14, wherein the patient is diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.
16. Larotrectinib, a pharmaceutically acceptable salt thereof, or a combination according to any one of embodiments 1 to 15, wherein the patient is not being concurrently treated with a strong CYP3A4 inducer.

1. Larotrectinib, a pharmaceutically acceptable salt thereof, or a combination thereof for use in a method for treating a patient having a cancer characterized by a neurotrophic receptor kinase gene fusion, wherein the patient is being concurrently treated with a moderate CYP3A4 inducer, said method comprising:
   administering larotrectinib, a pharmaceutically acceptable salt thereof, or a combination thereof, to the patient with a 1.5-2.5 fold increased daily dose of larotrectinib, pharmaceutically acceptable salt thereof, or combination thereof to the patient compared to a standard dose.
2. Larotrectinib, a pharmaceutically acceptable salt thereof, or a combination thereof according to embodiment 1, wherein a 2 fold increased daily dose of larotrectinib, pharmaceutically acceptable salt thereof, or combination thereof is administered.
3. Larotrectinib, a pharmaceutically acceptable salt thereof, or a combination thereof according to embodiment 1 or 2, wherein the daily dose is equivalent to 250-500 mg of the free base.
4. Larotrectinib, a pharmaceutically acceptable salt thereof, or a combination thereof according to any one of embodiments 1 to 3, wherein the patient being concurrently treated with a moderate CYP3A4 inducer receives the moderate CYP3A4 inducer within 12 hours of receiving the increased dose of larotrectinib, or pharmaceutically acceptable salt thereof.
5. Larotrectinib, a pharmaceutically acceptable salt thereof, or a combination thereof according to any one of embodiments 1 to 4, wherein the patient being concurrently treated with a moderate CYP3A4 inducer receives the moderate CYP3A4 inducer within 12 hours prior to receiving the increased dose of larotrectinib, or pharmaceutically acceptable salt thereof.
6. Larotrectinib, a pharmaceutically acceptable salt thereof, or a combination thereof according to any one of embodiments 1 to 5, wherein the patient being concurrently treated with a moderate CYP3A4 inducer receives the moderate CYP3A4 inducer within 12 hours after receiving the increased dose of larotrectinib, or pharmaceutically acceptable salt thereof.
7. Larotrectinib, a pharmaceutically acceptable salt thereof, or a combination thereof according to any one of embodiments 1 to 6, wherein the administration of the increased dose of larotrectinib, or pharmaceutically acceptable salt thereof, produces a larotrectinib plasma concentration (AUC0-inf) that is within 50% of the plasma concentration in a patient that receives a daily dose of larotrectinib or pharmaceutically acceptable in an amount that corresponds to a daily dose of 200 mg larotrectinib free base, wherein the patient is not being concurrently treated with a moderate CYP3A4 inducer.
8. Larotrectinib, a pharmaceutically acceptable salt thereof, or a combination thereof according to any one of embodiments 1 to 7, wherein the administration of the increased dose of larotrectinib, pharmaceutically acceptable salt thereof, or combination thereof does not produce an increase in CTCAE Grade 3 events,
   wherein the increase is relative to when the patient is not being concurrently treated with a moderate CYP3A4 inducer, and the patient receives a daily dose of larotrectinib or pharmaceutically acceptable salt in an amount that corresponds to a daily dose of 200 mg larotrectinib free base.
9. Larotrectinib, a pharmaceutically acceptable salt thereof, or a combination thereof according to any one of embodiments 1 to 8, wherein the administration of the increased dose of larotrectinib, or pharmaceutically acceptable salt thereof, does not produce an increase in CTCAE Grade 4 events,
   wherein the increase is relative to when the patient is not being concurrently treated with a moderate CYP3A4 inducer, and the patient receives a daily dose of larotrectinib or pharmaceutically acceptable salt in an amount that corresponds to a daily dose of 200 mg larotrectinib base.
10. Larotrectinib, a pharmaceutically acceptable salt thereof, or a combination thereof according to any one of embodiments 1 to 9, wherein the increased dose of larotrectinib, pharmaceutically acceptable salt thereof, or combination thereof, comprises a sulfate salt.
11. Larotrectinib, a pharmaceutically acceptable salt thereof, or a combination thereof according to any one of embodiments 1 to 10, wherein the increased dose of larotrectinib, pharmaceutically acceptable salt thereof, or combination thereof, comprises a sulfate salt having the formula. 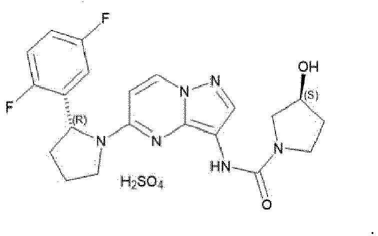
12. Larotrectinib, a pharmaceutically acceptable salt thereof, or a combination thereof according to any one of embodiments 1 to 11, wherein the increased dose of larotrectinib, pharmaceutically acceptable salt thereof, or combination thereof, is administered in a liquid formulation prepared from a larotrectinib sulfate.
13. Larotrectinib, a pharmaceutically acceptable salt thereof, or a combination thereof according to any one of embodiments 1 to 12, wherein the increased dose corresponds to a daily dose of larotrectinib free base between 250-500 mg.
14. Larotrectinib, a pharmaceutically acceptable salt thereof, or a combination thereof according to any one of embodiment 1 to 13, wherein the increased dose corresponds to a daily dose of 400 mg larotrectinib free base.
15. Larotrectinib, a pharmaceutically acceptable salt thereof, or a combination thereof according to any one of embodiments 1 to 14, wherein the increased dose corresponds to a daily dose of larotrectinib free base between 250-500 mg/m² if the patient has a body surface area less than 1 m².
16. Larotrectinib, a pharmaceutically acceptable salt thereof, or a combination thereof according to embodiment 15, wherein the increased dose corresponds to a daily dose of 400 mg/m² larotrectinib free base.
17. Larotrectinib, a pharmaceutically acceptable salt thereof, or a combination thereof according to any one of embodiments 1 to 16, wherein the increased dose is administered in two equal doses.
18. Larotrectinib, a pharmaceutically acceptable salt thereof, or a combination thereof according to any one of embodiments 1 to 17, wherein the increased dose is administered in two separate dosing events a day.
19. Larotrectinib, a pharmaceutically acceptable salt thereof, or a combination thereof according to any one of embodiments 1 to 18, wherein the separate dosing events are separated by at least 4 hours.
20. Larotrectinib, a pharmaceutically acceptable salt thereof, or a combination thereof according to any one of embodiments 1 to 19, wherein the neurotrophic receptor kinase gene fusion comprises one or more of ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.
21. Larotrectinib, a pharmaceutically acceptable salt thereof, or a combination thereof according to any one of embodiments 1 to 20, wherein the cancer characterized by a neurotrophic receptor kinase gene fusion comprises one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.
22. Larotrectinib, a pharmaceutically acceptable salt thereof, or a combination thereof according to any one of embodiments 1 to 21, wherein the moderate CYP3A4 inducer comprises a steroid.
23. Larotrectinib, a pharmaceutically acceptable salt thereof, or a combination according to any one of embodiments 1 to 22, wherein moderate CYP3A4 inducer comprises a steroid selected from the group consisting of dexamethasone, progesterone, norethindrone, estradiol, and combinations thereof.
24. Larotrectinib, a pharmaceutically acceptable salt thereof, or a combination thereof according to any one of embodiments 1 to 23, wherein the moderate CYP3A4 inducer comprises armodafinil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, St. John's wort, vemurafenib, zanubrutinib, or a combination thereof.
25. Larotrectinib, a pharmaceutically acceptable salt thereof, or a combination thereof according to any one of embodiments 1 to 24, wherein the patient is diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.
26. Larotrectinib, a pharmaceutically acceptable salt thereof, or a combination thereof according to any one of embodiments 1 to 25, wherein the patient is not being concurrently treated with a strong CYP3A4 inducer.

### EXAMPLES

The following examples are for the purpose of illustration of the invention only and are not intended to limit the scope of the present invention in any manner whatsoever.

The effect of different CYP3A4 inhibitors and inducers on the pharmacokinetics of larotrectinib was evaluated using an *in silico* model. The following drugs were included in the study:

| Drug | CYP3A4 Effect |
|---|---|
| Itraconazole | Strong CYP3A4 inhibitor |
| Fluconazole | Moderate CYP3A4 inhibitor |
| Diltiazem | Moderate CYP3A4 inhibitor |
| Cimetidine | Weak CYP3A4 inhibitor |
| Rifampicin* | Strong CYP3A4 inducer |
| Efavirenz | Moderate CYP3A4 induced |

| | |
|---|---|
| * rifampicin is alternatively known as rifampin. | |

The model was correlated by comparison of simulated and observed plasma concentrations in both healthy subjects and cancer patients for midazolam, testosterone, and several of the tested drugs, including larotrectinib alone, larotrectinib + intraconazole, larotrectinib + rifampicin. The following virtual trials were conducted:
12 healthy subjects (50% female) aged 20 to 55 years receiving larotrectinib (100 mg BID) for 14 days in the absence of itraconazole and coadminstered with itraconazole (tablets in fed state; 200 mg QD).

12 healthy subjects (50% female) aged 20 to 55 years receiving larotrectinib (100 mg BID) for 14 days in the absence of fluconazole and coadminstered with fluconazole (400 mg loading dose followed by 200 mg QD).

12 healthy subjects (50% female) aged 20 to 55 years receiving larotrectinib (100 mg BID) for 14 days in the absence of diltiazem and coadminstered with diltiazem (60 mg TID).

12 healthy subjects (50% female) aged 20 to 55 years receiving larotrectinib (100 mg BID) for 14 days in the absence of cimetidine and coadminstered with cimetidine (400 mg BID).

12 healthy subjects (50% female) aged 20 to 55 years receiving larotrectinib (100 mg BID) for 14 days in the absence of rifampicin and coadminstered with rifampicin (600 mg QD).

12 healthy subjects (50% female) aged 20 to 55 years receiving larotrectinib (100 mg BID) for 14 days in the absence of efavirenz and coadminstered with efavirenz (600 mg QD).

12 patients (36% female) aged 28 to 82 years receiving larotrectinib (100 mg BID) for 14 days in the absence of itraconazole and coadminstered with itraconazole (tablets in fed state; 200 mg QD).

12 patients (36% female) aged 28 to 82 years receiving larotrectinib (100 mg BID) for 14 days in the absence of fluconazole and coadminstered with fluconazole (loading dose of 400 mg followed by 200 mg QD).

12 patients (36% female) aged 28 to 82 years receiving larotrectinib (100 mg BID) for 14 days in the absence of diltiazem and coadminstered with diltiazem (60 mg TID).

12 patients (36% female) aged 28 to 82 years receiving larotrectinib (100 mg BID) for 14 days in the absence of cimetidine and coadminstered with cimetidine (400 mg BID).

12 patients (36% female) aged 28 to 82 years receiving larotrectinib (100 mg BID) for 14 days in the absence of rifampicin and coadminstered with rifampicin (600 mg QD).

12 patients (36% female) aged 28 to 82 years receiving larotrectinib (100 mg BID) for 14 days in the absence of efavirenz and coadminstered with efavirenz (600 mg QD).

A comparison was made between mean simulated and observed plasma concentrations of larotrectinib following a single oral dose of 100 mg larotrectinib in the absence of and co-administered with itraconazole on the 5th day of 7 days of dosing (200 mg QD). Simulated Cₘₐₓ and AUC and corresponding ratios for larotrectinib are shown below. The predicted changes in exposure of larotrectinib were consistent with observed data (within 1.27-fold) as indicated by the Cₘₐₓ and AUC ratios.

| | **Control** | | **Plus itraconazole** | | **Ratio** | |
|---|---|---|---|---|---|---|
| | **Cₘₐₓ (ng/mL)** | **AUC (ng/mL*h)** | **Cₘₐₓ (ng/mL)** | **AUC (ng/mL*h)** | **Cₘₐₓ** | **AUC** |
| Predicted | 550 | 1453 | 1215 | 6531 | 2.21 (2.1,4 2.29) | 4.49 (4.31, 4.69) |
| Observed | 497 | 1280 | 1395 | 5521 | 2.81 (2.26, 3.49) | 4.31 (3.76, 4.95) |
| P/O | 1.11 | 1.14 | 0.87 | 1.18 | 0.79 | 1.04 |

A comparison was made between mean simulated and observed plasma concentrations of larotrectinib following a single oral dose of 100 mg larotrectinib in the absence of and co-administered with rifampicin on the 10th day of 11 days of dosing (600 mg QD). Simulated Cₘₐₓ and AUC and corresponding ratios for larotrectinib are shown below.

The predicted changes in exposure of larotrectinib were reasonably consistent with observed data (within 1.32-fold) as indicated by the Cₘₐₓ and AUC ratios. However, it should be noted that the Cₘₐₓ and AUC ratios for at least 5 of the 10 virtual trials were within 1.25-fold of the observed data.

| | **Control** | | **Plus rifampicin** | | **Ratio** | |
|---|---|---|---|---|---|---|
| | **Cₘₐₓ (ng/mL)** | **AUC (ng/mL*h)** | **Cₘₐₓ (ng/mL)** | **AUC (ng/mL*h)** | **Cₘₐₓ** | **AUC** |
| Predicted | 523 | 1426 | 117 | 216 | 0.22 (0.21, 0.24) | 0.15 (0.14, 0.16) |
| Observed | 435 | 1343 | 133 | 259 | 0.29 (0.23, 0.37) | 0.19 (0.16, 0.24) |
| P/O | 1.20 | 1.06 | 0.88 | 0.83 | 0.76 | 0.79 |

Mean predicted plasma concentrations of larotrectinib following multiple oral doses of larotrectinib (100 mg BID) in the absence of and co-administered with itraconazole (200 mg QD) over 14 days are shown below:

| | **Control** | | **Plus itraconazole** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC_{(0,T)}** |
| Predicted | 701 | 2074 | 1444 | 7515 | 2.06 (1.98,2.14) | 3.62 (3.40,3.86) |

Mean predicted plasma concentrations of larotrectinib following multiple oral doses of larotrectinib (100 mg BID) in the absence of and co-administered with fluconazole (loading dose of 400 mg followed by 200 mg QD) over 14 days are shown below:

| | Control | | Plus fluconazole | | Ratio | |
|---|---|---|---|---|---|---|
| | Cₘₐₓ (ng/mL) | AUC(_{0.T}) (ng/mL^{∗}h) | Cₘₐₓ (ng/mL) | AUC(_{0,T}) (ng/mL^{∗}h) | Cₘₐₓ | AUC_{(0,T)} |
| Predicted | 695 | 2074 | 1161 | 5017 | 1.67 (1.63, 1.72) | 2.42 (2.33, 2.52) |

Mean predicted plasma concentrations of larotrectinib following multiple oral doses of larotrectinib (100 mg BID) in the absence of and co-administered with diltiazem (60 mg TID) over 14 days are shown below:

| | **Control** | | **Plus diltiazem** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0.T)} (ng/mL*h)** | **Cₘₐₓ** | **AUC_{(0,T)}** |
| Predicted | 701 | 2074 | 967 | 3562 | 1.38 (1.35, 1.41) | 1.72 (1.66, 1.78) |

Mean predicted plasma concentrations of larotrectinib following multiple oral doses of larotrectinib (100 mg BID) in the absence of and co-administered with cimetidine (400 mg BID) over 14 days are shown below:

| | **Control** | | **Plus cimetidine** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC_{(0,T)}** |
| Predicted | 670 | 2074 | 816 | 2728 | 1.22 (1.20, 1.23) | 1.32 (1.30, 1.33) |

Mean predicted plasma concentrations of larotrectinib following multiple oral doses of larotrectinib (100 mg BID) in the absence of and co-administered with rifampicin (600 mg QD) over 14 days are shown below:

| | **Control** | | **Plus rifampicin** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC_{(0,T)}** |
| Predicted | 695 | 2071 | 139.7 | 255 | 0.20 (0.19,0.22) | 0.12 (0.11, 0.13) |

Mean predicted plasma concentrations of larotrectinib following multiple oral doses of larotrectinib (100 mg BID) in the absence of and co-administered with efavirenz (600 mg QD) over 14 days are shown below:

| | Control | | Plus rifampicin | | Ratio | |
|---|---|---|---|---|---|---|
| | Cₘₐₓ (ng/mL) | AUC_{(0,T)} (ng/mL^{∗}h) | Cₘₐₓ (ng/mL) | AUC{_{O},ₗ) (ng/mL^{∗}h) | C_{mag} | AUC_{(0,T)} |
| Predicted | 670 | 2074 | 276.8 | 580 | 0.41 (0.39, 0.44) | 0.28 (0.26, 0.30) |

100 mg BID larotrectinib was administered in the absence of any CYP3A4 perpetrators. The observed Cₘₐₓ and AUC values were 624 ng/mL and 1590 ng/mL*h, respectively.

Predicted Cₘₐₓ and AUC values for larotrectinib after multiple oral doses of larotrectinib (10 to 100 mg BID) in the presence of itraconazole (200 mg QD)

| **Dose (mg)** | **Cmax (plus itraconazole) (ng/mL)** | **AUC (plus itraconazole) (ng/mL.h)** |
|---|---|---|
| 10 | 108 | 568 |
| 20 | 225 | 1262 |
| 30 | 348 | 2031 |
| 40 | 475 | 2832 |
| 50 | 602 | 3649 |
| 60 | 730 | 4468 |
| 70 | 857 | 5289 |
| 80 | 985 | 6112 |
| 90 | 1113 | 6934 |
| 100 | 1241 | 7757 |

Predicted Cₘₐₓ and AUC values for larotrectinib after multiple oral doses of larotrectinib (50 to 100 mg BID) in the presence of fluconazole (loading dose of 400 mg followed by 200 mg QD)

| **Dose (mg)** | **Cmax (plus fluconazole) (ng/mL)** | **AUC (plus fluconazole) (ng/mL.h)** |
|---|---|---|
| 50.0 | 406 | 1657 |
| 62.5 | 520 | 2171 |
| 75.0 | 637 | 2719 |
| 87.5 | 759 | 3307 |
| 100.0 | 883 | 3925 |

Predicted exposures of larotrectinib following 14 days of dosing at 100 to 500 mg BID in the presence of rifampicin (600 mg QD) or efavirenz (600 mg QD) are shown below: In the presence of rifampicin, a dose increase of 100 mg BID to 500 mg BID of larotrectinib is predicted to most closely match dosing without rifampicin. In the presence of efavirenz, a dose increase of 100 mg BID to 200 mg BID of larotrectinib is predicted to most closely match dosing without efavirenz.

Predicted Cₘₐₓ and AUC values for larotrectinib after multiple oral doses of larotrectinib (100 to 500 mg BID) in the presence of rifampicin (600 mg QD)

| **Dose (mg)** | **Cmax (plus rifampicin) (ng/mL)** | **AUC (plus rifampicin) (ng/mL.h)** |
|---|---|---|
| 100 | 100 | 183 |
| 200 | 222 | 411 |
| 300 | 366 | 682 |
| 400 | 529 | 994 |
| 500 | 713 | 1349 |

Predicted Cₘₐₓ and AUC values for larotrectinib after multiple oral doses of larotrectinib (100 to 500 mg BID) in the presence of efavirenz (600 mg QD):

| **Dose (mg)** | **Cmax (plus efavirenz) (ng/mL)** | **AUC (plus efavirenz) (ng/mL.h)** |
|---|---|---|
| 100 | 271 | 572 |
| 200 | 602 | 1306 |
| 300 | 993 | 2229 |
| 400 | 1449 | 3383 |
| 500 | 1978 | 4822 |

In patients with elevated cytokines, CYP3A4 levels are suppressed. When given a drug that reduces the level of cytokines, the amount of CYP3A4 increases over time. Prior to taking larotrectinib, the cancer patients may be in a suppressed state as a consequence of elevated cytokines. After taking the drug, the reduction in cytokines which occurs as a result of resolution of the disease leads to a reversal of the suppression of CYP3A4. This is opposed by the CYP3A4 inactivation. To address both scenarios, modeling with auto-inhibition of CYP3A4 and without autoinhibition was applied.

Mean predicted plasma concentrations of larotrectinib following multiple oral doses of larotrectinib (100 mg BID) in the absence of and co-administered with cimetidine (400 mg BID) over 14 days are shown below:

| | **Control** | | **Plus cimetidine** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC**_{(0,T)} |
| Inactivation | 732 | 3207 | 905 | 4373 | 1.24 (1.22, 1.25) | 1.36 (1.34, 1.38) |
| No inactivation | 527 | 1899 | 673 | 2597 | 1.28 (1.26, 1.29) | 1.37 (1.35, 1.38) |

Mean predicted plasma concentrations of larotrectinib following multiple oral doses of larotrectinib (100 mg BID) in the absence of and co-administered with rifampicin (600 mg QD) over 14 days are shown below:

| | **Control** | | **Plus rifampicin** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC_{(0,T)}** |
| Inactivation | 761 | 3204 | 139 | 314 | 0.18 (0.17, 0.20) | 0.10 (0.09, 0.11) |
| No inactivation | 548 | 1899 | 116 | 258 | 0.21 (0.20, 0.23) | 0.14 (0.12, 0.15) |

Mean predicted plasma concentrations of larotrectinib following multiple oral doses of larotrectinib (100 mg BID) in the absence of and co-administered with efavirenz (600 mg QD) over 14 days are shown below:

| | **Control** | | **Plus efavirenz** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC_{(0,T)}** |
| Inactivation | 732 | 3207 | 256 | 662 | 0.35 (0.33, 0.37) | 0.21 (0.19, 0.22) |
| No inactivation | 527 | 1899 | 212 | 523 | 0.40 (0.38, 0.42) | 0.28 (0.26, 0.29) |

Geometric mean predicted Cₘₐₓ and AUC values and corresponding ratios for larotrectinib (90% CI) after a multiple oral dose of larotrectinib (100 mg BID) in the absence of efavirenz (control) and presence of efavirenz (600 mg QD)

| | **Control** | | **Plus efavirenz** | | **Ratio** | |
|---|---|---|---|---|---|---|
| | **Cₘₐₓ (ng/mL)** | **AUC_{(0,T)} (ng/mL*h)** | **Cₘₐₓ (ng/mL)** | **AUC_{(0,T)} (ng/mL*h)** | **Cₘₐₓ** | **AUC_{(0,T)}** |
| Inactivation | 732 | 3207 | 256 | 662 | 0.35 (0.33, 0.37) | 0.21 (0.19, 0.22) |
| No inactivation | 527 | 1899 | 212 | 523 | 0.40 (0.38, 0.42) | 0.28 (0.26, 0.29) |

After a single oral dose of 100 mg larotrectinib co-administered with itraconazole on the 5th day of 7 days of dosing (200 mg QD) in healthy subjects, simulated exposures were within 1.25-fold of observed data; the predicted and observed Cₘₐₓ and AUC ratios were 2.21 and 4.49, respectively, and 2.81 and 4.31, respectively. Furthermore, following coadministration of larotrectinib with rifampicin (600 mg QD), the observed reduction in exposure of larotrectinib (71% and 81% Cₘₐₓ and AUC, respectively were accurately predicted (78% and 85% Cₘₐₓ and AUC, respectively). At this point, the model for larotrectinib was considered to be verified with respect to CYP3A4-mediated metabolism. The magnitude of interaction was similar irrespective of whether CYP3A4 inactivation was considered or not. Furthermore, the magnitude of interactions was similar for both healthy subjects and cancer patients.

Geometric mean predicted Cₘₐₓ and AUC ratios for larotrectinib (90% CI) in healthy subjects after dosing of larotrectinib (100 mg BID) with CYP3A4 perpetrators at steady state:

| | Ratio | |
|---|---|---|
| | Cₘₐₓ | AUC_{(0,T)} |
| Itraconazole | 206 (1.98, 2.14) | 3.62 (3.40, 3.86) |
| Strong CYP3A4 inhibitor | | |
| Fluconazole | 1.56 (1.52, 1.59) | 2.12 (2.04, 2.79) |
| Moderate CYP3A4 inhibitor | | |
| Diltiazem | 138 (1.35, 1.41) | 1.72 (1.66, 1.78) |
| Moderate CYP3A4 inhibitor | | |
| Cimetidine | 1.22 (1 20, 1.23) | 1.32 (1.30, 1.33) |
| Weak CYP3A4 inhibitor | | |
| Rifampicin | 0.20 (0.19, 0.22) | 0.12 (0.11, 0.13) |
| Strong CYP3A4 inducer | | |
| Efavirenz | 0.41 (0.39, 0.44) | 0.28 (0.26, 0.30) |
| Moderate CYP3A4 inducer | | |

Geometric mean predicted Cₘₐₓ and AUC ratios for larotrectinib (90% CI) in cancer patients after dosing of larotrectinib (100 mg BID) with CYP3A4 perpetrators at steady state

| | | **Ratio** | |
|---|---|---|---|
| | | **Cₘₐₓ** | **AUC_{(0,T)}** |
| Itraconazole | Inactivation | 2.41 (4.07, 4.72) | 4.38 (2.31, 2..53) |
| | No inactivation | 2.78 (2.68, 2.88) | 5.29 (5.01, 5.59) |
| Fluconazole | Inactivation | 1.83 (1.78, 1.88) | 2.75 (2.64, 2.86) |
| | No inactivation | 1.86 (1.83, 1.90) | 2.72 (2_66, 2.78) |
| Diltiazem | Inactivation | 1.45 (1.43, 1.47) | 1.88 (1.83, 1.94) |
| | No inactivation | 1.78 (1.74, 1.82) | 2.55 (2.46, 2.66) |
| Cimetidine | Inactivation | 1.24 (1.22, 1.25) | 1.36 (1.34, 1.38) |
| | No inactivation | 1.28 (1.26, 1.29) | 1.37 (1.35,1.38) |
| Rifampicin | Inactivation | 0.18 (0.17, 0.20) | 0.10 (0.09, 0.11) |
| | No inactivation | 0.21 (0.20, 0.23) | 0.14 (0.12, 0.15) |
| Efavirenz | Inactivation | 0.35 (0.33, 0_37) | 0.21 (0.19, 0.22) |
| | No inactivation | 0.4 (0.38,0.42) | 0.28 (0.26, 0.29) |

The compositions and methods of the appended claims are not limited in scope by the specific compositions and methods described herein, which are intended as illustrations of a few aspects of the claims, and any compositions and methods that are functionally equivalent are intended to fall within the scope of the claims. Various modifications of the compositions and methods, in addition to those shown and described herein, are intended to fall within the scope of the appended claims. Further, while only certain representative compositions and method steps disclosed herein are specifically described, other combinations of the compositions and method steps also are intended to fall within the scope of the appended claims, even if not specifically recited. Thus, a combination of steps, elements, components, or constituents may be explicitly mentioned herein or less, however, other combinations of steps, elements, components, and constituents are included, even though not explicitly stated. The term "comprising" and variations thereof, as used herein, is used synonymously with the term "including" and variations thereof and are open non-limiting terms. Although the terms "comprising" and "including" have been used herein to describe various embodiments, the terms "consisting essentially of" and "consisting of" can be used in place of "comprising" and "including" to provide for more specific embodiments of the invention and are also disclosed. Other than in the examples, or where otherwise noted, all numbers expressing quantities of ingredients, reaction conditions, and so forth used in the specification and claims are to be understood at the very least and not as an attempt to limit the application of the doctrine of equivalents to the scope of the claims, to be construed in light of the number of significant digits and ordinary rounding approaches.

## Claims

1. Larotrectinib, a pharmaceutically acceptable salt thereof, or a combination thereof for use in a method for treating a patient having a cancer **characterized by** a neurotrophic receptor kinase gene fusion, wherein the patient has received and will receive a moderate CYP3A4 inducer, said method comprising:
administering larotrectinib, a pharmaceutically acceptable salt thereof, or a combination thereof, to the patient with a 1.5-2.5 fold daily dose of Larotrectinib, pharmaceutically acceptable salt thereof, or combination thereof to the patient compared to a standard dose.

2. Larotrectinib, a pharmaceutically acceptable salt thereof, or a combination thereof according to claim 1, wherein a 2 fold daily dose of larotrectinib, pharmaceutically acceptable salt thereof, or combination thereof is administered.

3. Larotrectinib, a pharmaceutically acceptable salt thereof, or a combination thereof according to claim 1 or 2, wherein the larotrectinib, pharmaceutically acceptable salt thereof, or combination thereof, comprises a sulfate salt having the formula.

4. Larotrectinib, a pharmaceutically acceptable salt thereof, or a combination thereof according to claim 1, 2, or 3, wherein the larotrectinib, pharmaceutically acceptable salt thereof, or combination thereof, is administered in a liquid formulation prepared from a larotrectinib sulfate.

5. Larotrectinib, a pharmaceutically acceptable salt thereof, or a combination thereof according to any one of claims 1 to 4, wherein the larotrectinib, pharmaceutically acceptable salt thereof, or combination thereof is administered with a daily dose that is equivalent to 250-500 mg of the free base.

6. Larotrectinib, a pharmaceutically acceptable salt thereof, or a combination thereof according to any one of claims 1 to 5, wherein the larotrectinib, pharmaceutically acceptable salt thereof, or combination thereof is administered with a daily dose that is equivalent to 400 mg of the free base.

7. Larotrectinib, a pharmaceutically acceptable salt thereof, or a combination thereof according to any one of claims 1 to 6, wherein the larotrectinib, pharmaceutically acceptable salt thereof, or combination thereof is administered in two equal doses.

8. Larotrectinib, a pharmaceutically acceptable salt thereof, or a combination thereof according to any one of claims 1 to 7, wherein the larotrectinib, pharmaceutically acceptable salt thereof, or combination thereof is administered with a daily dose that is equivalent to 250-500 mg/m² of the free base if the patient has a body surface area less than 1 m².

9. Larotrectinib, a pharmaceutically acceptable salt thereof, or a combination thereof according to claim 8, wherein the larotrectinib, pharmaceutically acceptable salt thereof, or combination thereof is administered with a daily dose that is equivalent to 400 mg/m² of the free base.

10. Larotrectinib, a pharmaceutically acceptable salt thereof, or a combination thereof according to any one of claims 1 to 9, wherein the neurotrophic receptor kinase gene fusion comprises one or more of ETV6-NTRK3, TPM3-NTRK1, LMNA-NTRK1, inferred ETV6-NTRK3, IRF2BP2-NTRK1, SQSTIM1-NTRK1, PDE4DIP-NTRK1, PPL-NTRK1, STRN-NTRK1, TPM4-NTRK3, TPR-NTRK1, TRIM63-NTRK1, CTRC-NTRK1, GON4L-NTRK1, or PLEKHA6-NTRK1.

11. Larotrectinib, a pharmaceutically acceptable salt thereof, or a combination thereof according to any one of claims 1 to 10, wherein the cancer **characterized by** a neurotrophic receptor kinase gene fusion comprises one or more of soft tissue sarcoma, salivary gland cancer, infantile fibrosarcoma, thyroid cancer, lung cancer, melanoma, colon cancer, gastrointestinal stromal tumor, cholangiocarcinoma, cancer of the appendix, breast cancer, or pancreatic cancer.

12. Larotrectinib, a pharmaceutically acceptable salt thereof, or a combination according to any one of claims 1 to 11, wherein the moderate CYP3A4 inducer comprises a steroid.

13. Larotrectinib, a pharmaceutically acceptable salt thereof, or a combination according to any one of claims 1 to 12, wherein moderate CYP3A4 inducer comprises a steroid selected from the group consisting of dexamethasone, progesterone, norethindrone, estradiol, and combinations thereof.

14. Larotrectinib, a pharmaceutically acceptable salt thereof, or a combination according to any one of claims 1 to 13, wherein the moderate CYP3A4 inducer comprises armodafinil, bexarotene, bosentan, cenobamate, dabrafenib, dexamethasone, dipyrone, efavirenz, elagolix, estradiol, norethindrone, eslicarbazepine, etravirine, lorlatinib, mitapivat, mobocertinib, modafinil, nafcillin, oxcarbazepine, pexidartinib, pioglitazone, pleconaril, primidone, troglitazone, terbinafine, rosiglitazone, rifabutin, rifapentine, rufinamide, sotorasib, St. John's wort, vemurafenib, zanubrutinib, or a combination thereof.

15. Larotrectinib, a pharmaceutically acceptable salt thereof, or a combination according to any one of claims 1 to 14, wherein the patient is diagnosed with cutaneous T-cell lymphoma pulmonary arterial hypertension, partial onset seizures, HIV, pain secondary to endometriosis, hemolytic anemia, narcolepsy, staphylococcal infection, staphylococcal endocarditis, mycobacterium avium complex (MAC) disease, tuberculosis, Lennox-Gastaut syndrome, fungal infection, or diabetes.

16. Larotrectinib, a pharmaceutically acceptable salt thereof, or a combination according to any one of claims 1 to 15, wherein the patient is not being concurrently treated with a strong CYP3A4 inducer.
